# EUROPEAN PATENT APPLICATION

(11) **EP 4 248 838 A1**
(43) Date of publication of application: **27.09.2023**
(21) Application number: 21894352.0
(22) Date of filing: 07.10.2021
(51) Int. Cl.: A61B 3/028

(54) **OPTOMETRY CONTROL PROGRAM AND SUBJECTIVE OPTOMETRY SYSTEM**

(30) Priority: 20.11.2020 JP 2020193730; 04.12.2020 JP 2020201880
(71) Applicant: Nidek Co., Ltd., Gamagori-shi, Aichi, 443-0038 (JP)
(72) Inventor: HORINO Taeko, Gamagori-shi Aichi 443-0038 (JP); TERABE Hirohisa, Gamagori-shi Aichi 443-0038 (JP)
(74) Representative: Viering, Jentschura & Partner mbB Patent- und Rechtsanwälte
(86) International application number: PCT/JP2021/037111
(87) International publication number: WO 2022/107477

(57) **Abstract**

A first information processing device executes a self-optometry progress step, a response acquisition step, a correction value storing step, and a self-optometry assistance step. At the self-optometry progress step, a presentation instruction signal for instructing a presentation operation to present the visual target to the examinee is outputted to the subjective optometry device in accordance with a progress procedure for a self-optometry that automatically proceeds. At the response acquisition step, a response from the examinee who visually recognized the presented visual target is acquired. At the correction value storing step, a correction value of the optical characteristic of the subject eye is stored. At the self-optometry assistance step, an assistance operation to assist in a progress of the self-optometry is executed when a problem occurs during the progress of the self-optometry.

## Description

### Technical Field

The present disclosure relates to an optometry control program executed by a subjective optometry system and the subjective optometry system.

### Background Art

A subjective optometry device has been known for measuring optical characteristics such as refractive power of a subject eye by presenting an examination target to the subject eye through an optical element in front of the subject eye. For example, the subjective optometry device disclosed in Patent Document 1 includes a refractive power measuring unit, an examination target presenting unit, and a controller. The refractive power measuring unit selects an optical element to be placed through an examination window from among a plurality of optical elements included in a correction optical system by a driver that switches the optical elements. The examination target presenting unit switches the examination targets each of which is presented to the subject eye. The controller detects a user operation on an operation panel and transmits a driving signal to the refractive power measuring unit and the examination target presenting unit based on the detected user operation.

### Citation List

### Patent Literature

Patent Literature 1: Japanese Patent Application Laid-Open No. 2020-018712

### Summary of Invention

In a conventional subjective optometry device, it was necessary for an examiner to be present with the examinee and input instructions for the next operation of the subjective optometry device based on responses from the examinee who visually recognized a visual target. Therefore, it was difficult for such a conventional subjective optometry device to execute subjective optometry smoothly while reducing the burden on the examiner.

One objective of the present disclosure is to provide a subjective optometry control program and a subjective optometry system that enable it possible to perform a subjective optometry more smoothly.

An optometry control program according to one aspect of the present disclosure is for a subjective optometry system including a subjective optometry device and a first information processing device connected to the subj ective optometry device. The subj ective optometry device includes a correction optical system that changes an optical characteristic of a visual target light flux presented to a subject eye and a visual target presentation unit that is configured to present a visual target to the subject eye. The subjective optometry device subjectively measures an optical characteristic of the subject eye. The program is executed by the first information processing device and comprises a self-optometry application program that realizes an application for causing a self-optometry to proceed automatically based on a response from an examinee. The self-optometry application program, when executed by a controller of the first information processing device, causes the first information processing device to perform: a self-optometry progress step of outputting, to the subjective optometry device, a presentation instruction signal for performing a presentation operation to present the visual target to the examinee in accordance with a progress procedure for a self-optometry that automatically proceeds; a response acquisition step of acquiring the response from the examinee who viewed the presented visual target; a correction value storing step of storing a correction value of the optical characteristic of the subject eye, the correction value being acquired based on the response acquired at the response acquisition step, the visual target that was presented by the subjective optometry device when the response was acquired, and the optical characteristic of the visual target light flux; and a self-optometry assistance step of executing an assistance operation to assist in a progress of the self-optometry when a problem occurs during the progress of the self-optometry.

A subjective optometry system according to another aspect of the present disclosure includes: a subjective optometry device including a correction optical system that changes an optical characteristic of a visual target light flux presented to a subject eye and a visual target presentation unit that is configured to present a visual target to the subject eye, the subjective optometry device subjectively measuring an optical characteristic of the subject eye; and a first information processing device connected to the subjective optometry device. The first information processing device includes a self-optometry application program that causes a self-optometry to proceed automatically based on a response from an examinee. The self-optometry application program, when executed, performs: a self-optometry progress step of outputting, to the subjective optometry device, a presentation instruction signal for performing a presentation operation to present the visual target to the examinee in accordance with a progress procedure for the self-optometry that automatically proceeds; a response acquisition step of acquiring the response from the examinee who visually recognized the presented visual target; a correction value storing step of storing a correction value of the optical characteristic of the subject eye, the correction value being acquired based on the response acquired at the response acquisition step, the visual target that was presented by the subjective optometry device when the response was acquired, and the optical characteristic of the visual target light flux; and a self-optometry assistance step of executing an assistance operation to assist in a progress of the self-optometry when a problem occurs during the progress of the self-optometry.

According to the optometry control program and the subjective optometry system disclosed herein, a subject optometry can be executed performed smoothly.

The subjective optometry system disclosed in the present disclosure includes the subjective optometry device and the first information processing device. The subjective optometry device includes the correction optical system that changes an optical characteristic of a visual target light flux presented to the subject eye, and the target presentation unit that presents a visual target to the subject eye. The subjective optometry device is used to subjectively measure the optical characteristic of the subject eye. The first information processing device is an information processing device connected to the subjective optometry device (hereinafter, the first information processing device may also be referred to as a "connection device"). The optometry control program disclosed herein includes a self-optometry application program. The self-optometry application program is a program that realizes an application (a self-optometry application) that automatically proceed with an optometry based on responses input by the examinee.

The self-optometry application program executes a subjective optometry progress step, a response acquisition step, a correction value storage step, and a subjective optometry assistance step by the control unit of the first information processing device. In the subjective optometry progress step, the first information processing device sequentially outputs a plurality of presentation instruction signals to the subjective optometry device for presenting the visual target to the examinee in accordance with at least a progress procedure along which the subjective optometry automatically proceeds (i.e., along the progress procedure). It should be noted that in the subjective optometry progress step, the first information processing device may control the correction optical system and the target presentation unit to execute a plurality of examination items based on the progress procedure for the subjective optometry which automatically proceeds. At the response acquisition step, the first information processing device acquires responses inputted by the examinee who visually recognized the presented visual target. In the correction value storage step, the first information processing device stores a correction value of the optical characteristic of the subject eye, which is obtained based on the answers acquired at the response acquisition step and the optical characteristics of the presented visual target and of the visual target light flux presented by the subjective optometry device. In the subjective optometry assistance step, the first information processing device executes an assistance operation to assist in the auto-subjective optometry when a problem occurs during the auto-subjective optometry.

According to the subjective optometry system disclosed herein, even if an examiner does not proceed with the examination, an appropriate eye examination for an examinee can be performed by executing the self-optometry application. Furthermore, if a problem occurs during the self-optometry, the assistance operation for assisting in the self-optometry is executed. In other words, a function for assisting in the progress of the self-optometry is included in functions of the self-optometry application. Therefore, for example, during the execution of the self-optometry application, at least one of assistance operations for the self-optometry, such as providing an advice from the examiner, is executed. Thus, the subjective optometry can be properly performed with reduced burden on the examiner.

Note that various devices can be used as the first information processing device that executes the optometry control program. For example, a personal computer (PC) may be used as the first information processing device. Alternatively, a server, a mobile terminal, or a smartphone may be used as the first information processing device.

The first information processing device may be formed by combining two or more of a plurality of devices. For example, the first information processing device may be formed of a device, such as a personal computer, and a dedicated controller having a controlling unit and a memory. Of course, the dedicated controller itself may serve as the first information processing device by having functions of a device such as a personal computer equipped with a CPU.

The storage device for storing the optometry control program may be appropriately selected. For example, the optometry control program may be stored in a memory built into the first information processing device or in a detachable storage device for the first information processing device. The optometry control program may also be stored in a memory built into the above-described dedicated controller. Additionally, the optometry control program may be stored in multiple storage devices.

In the self-optometry assistance step, when a predetermined condition is met during the execution of the self-optometry, or when an instruction for executing the assistance operation is input, the assistance operation for assisting in the self-optometry may be executed. In this case, when the examinee is unable to perform the self-optometry due to certain circumstances, the assistance operation for the self-optometry is performed appropriately.

Note that the conditions in the self-optometry for executing the assistance operation may be appropriately selected. For example, the control unit may determine that the condition for executing the assistance operation is met when a predetermined time has elapsed without receiving an input of a response from the examinee. Alternatively, the control unit may determine that the condition for executing the assistance operation is met when a response is inappropriately input by the examinee. The method for determining whether the response is inappropriate may be appropriately selected. For example, the control unit may determine that the input response is inappropriate if the response does not meet a predetermined response condition (e.g., if a response different from candidate responses expected to receive from the examinee is input, if the same responses are input consecutively more than a predetermined number of times, or if the number of responses exceeds the number of responses expected to be input from the examinee).

The assistance operation executed at the self-optometry assistance step may include a call operation for calling for an examiner. In this case, when a problem occurs during the self-optometry, the examiner is called so that a subsequent examination can properly proceed.

The self-optometry assistance step may include a manual progress step in which a presentation instruction signal for advancing at least a part of the progress procedure is outputted to the subjective optometry device in response to receiving an instruction input from an examiner. In this case, the examiner may manually advance at least an examination step at which a problem occurred among the progress procedure that are planned to be performed during the self-optometry. Therefore, the optometry for the examinee can be more appropriately supported.

The self-optometry assistance step may include a correction value modification step to modify at least one of correction values stored during the self-optometry in response to receiving an instruction inputted by an examiner. In this case, for example, if a correction value that failed to be measured during the self-optometry exists, the correction value can be modified based on the examiner's decision. Therefore, the optometry for the examinee may smoothly proceeds.

The self-optometry assistance step may include a procedure omission step to omit at least a part of the progress procedure in response to receiving an instruction inputted by an examiner. In this case, the examiner can omit an unnecessary step from the predetermined steps. Therefore, the optometry for the examinee can proceed smoothly.

The method for inputting the instruction by the examiner to omit at least a part of the steps may be appropriately selected. For example, the examiner may input, into the first information processing device, a selection instruction for omitting a test from among the multiple tests executed in accordance with the progress procedure. In this case, the control unit of the first information processing device may omit the selected test from the tests. Also, the examiner may input, into the first information processing device, a timing for resuming the self-optometry, which is temporarily stopped, as a timing subsequent to the omitted step. In this case, the control unit of the first information processing device may omit a part of the steps by resuming the self-optometry from the inputted timing which is set after the omitted step.

The self-optometry assistance step may include a proxy response acquisition step. In the proxy response acquisition step, the control unit acquires, in response to an instruction inputted by an examiner, a response from an examinee who has visually recognized a visual target that was presented in accordance with the progress procedure at the self-optometry step. In this case, during the process in which visual targets are presented to the examinee in an order according to the progress procedure of the self-optometry, the examiner can hear the responses from the examinee who has recognized the visual target and enter the responses heard from the examinee. Therefore, even if the examinee is not familiar with a way of responding in the self-optometry, the examiner can assist in the self-optometry properly by entering the responses heard from the examinee on behalf of the examinee.

The self-optometry assistance step may include a continuous resumption step in which the self-optometry that has been temporarily stopped resumes from the next step after the step for which the optometry has already been completed (for example, a completed test). In this case, the self-optometry resumes from the next step after the step for which the optometry has already been completed. Therefore, even after the assistance for the self-optometry has been completed, the self-optometry can properly continue along the progress procedure.

The self-optometry assistance step may include a designated resumption step in which the self-optometry that has been temporarily stopped resumes from a step designated by the examiner (for example, a designated test). In this case, the examiner can resume the self-optometry from the step designated by him/her when a problem that occurred during the self-optometry is resolved, etc. Therefore, burden on the examiner for assisting in the optometry can be prevented from excessively increasing.

The progress procedure for the self-optometry may define multiple tests (examinations) performed on the examinee and an order in which the multiple tests are conducted. At the self-optometry progress step, multiple tests may be performed sequentially in accordance with the progress procedure. In this case, the multiple tests for the examinee can be done automatically and appropriately. Furthermore, the examiner may perform suitable assistance for tests that are determined by the examiner to require for assistance among the multiple tests (for example, a test where a proper correction value cannot be acquired in the self-optometry).

The self-optometry assistance step may include a step for displaying the progress procedure on a display unit. The display unit may be located in a facility where the examiner is waiting, may be located in a different facility than a facility where the self-optometry device is located, or may be located in the same facility where the self-optometry device is located. During the designated resumption step, an instruction for designating a step from which the self-optometry will resume (hereinafter referred to as a "resumption step") for resuming the self-optometry may be accepted through an operation on the progress procedure displayed on the display unit. In this case, the examiner can designate the resumption step to resume the self-optometry while properly recognizing the details and progress status of the progress procedure displayed on the display unit.

Note that a specific method for accepting the input of the resumption step through an operation on the displayed progress procedure can be appropriately selected. For example, a touch panel may be disposed in a display area of the display unit. In this case, the control unit may treat, as the resumption step designated by the examiner, the step (for example, a specific test) corresponding to the position on the touch panel where the touch panel is operated among the displayed progress procedure. Alternatively, a mouse or the like for moving a pointer within the display area of the display unit may be provided. In this case, the control unit may treat the step corresponding to the position of the pointer on the displayed progress procedure as the resumption step designated by the examiner.

The control unit may display, on the progress procedure, at least one of the step currently being executed or the step during which a problem occurred among the entire progress procedure (e.g., multiple tests included in the progress procedure). In this case, the examiner can properly recognize the progress status of the self-optometry system by the displayed progress procedure.

As described above, resuming the self-optometry that was temporarily stopped leads to obtaining various benefits. For example, in the cross-cylinder test, which is one of the tests performed by the subjective optometry system, two sets of dots are presented to an examinee. However, if the relative position between the examination window of the correction optical system and the subject eye is misaligned, the examinee would be able to recognize only one of the two sets of dots. In this case, the examinee would not be able to conduct the self-optometry. However, according to the present disclosure's subjective optometry system, while the self-optometry is temporarily stopped, the examiner can make an advice to the examinee to adjust the position of the subject eye relative to the examination window, for example, to assist in the progress of the cross-cylinder test. Then, the examinee can resume the self-optometry by himself/herself. Thus, resuming the temporarily stopped self-optometry leads to useful effects for both the examiner and the examinee.

The self-optometry assistance step may be executed based on an instruction inputted to the second information processing device that is connected to the first information processing device via a network. In this case, even if the examiner is located at a remote location away from the examinee, the examiner can properly assist the examinee in the self-optometry from the remote location by inputting instructions to the second information processing device.

If a problem occurs during the progress of the self-optometry (for example, if an instruction for the assistance operation is inputted), the control unit of the first information processing device may output, to the second information processing device, an instruction to execute a call operation to call for the examiner. In this case, even if the examiner is at a remote location away from the examinee, the examiner can easily recognize that a problem occurred during the progress of the self-optometry.

However, the self-optometry assistance step may be executed in response to instructions directly inputted to the first information processing device (for example, instructions inputted to the operation unit of the first information processing device). That is, the examiner located at the location where the subjective optometry device and the first information processing device are installed may input instructions to execute the self-optometry assistance step. In this case, the operation unit (examiner's controller) of the first information processing device used for inputting instructions by the examiner may be a dedicated controller provided in the subjective optometry device, or a general-purpose user interface such as a mouse or a tablet.

Furthermore, when the second information processing device is used, the number of the second information processing devices may be one or more. Similar to the first information processing device, various devices (such as PCs, mobile terminals, or smartphones) can be used as the second information processing device. The second information processing device may be connected, in advance, to the first information processing device when the self-optometry is performed, or may be connected to the first information processing device after a problem occurs during the self-optometry.

When the second information processing device is used, each of the control units of the first and second information processing devices may perform a remote conversation step to assist in conversation between the examiner and the examinee by transmitting and receiving at least audio data (which may include both audio and image data) between the examiner and the examinee. In this case, the examiner located remotely can appropriately assist in the self-optometry by recognizing the examinee's situation through the conversation.

### Brief Description of Drawings

Fig. 1 is a block diagram showing a configuration of a subjective optometry system 100.
Fig. 2 is a flowchart of an optometry control process executed by a first information processing device 2A.
Fig. 3 is a diagram showing an example of a self-optometry assistance screen in the embodiment.
Fig. 4 is a flowchart of a self-optometry assistance process executed during the optometry control process.

### Modes for Carrying Out the Invention

### (System configuration)

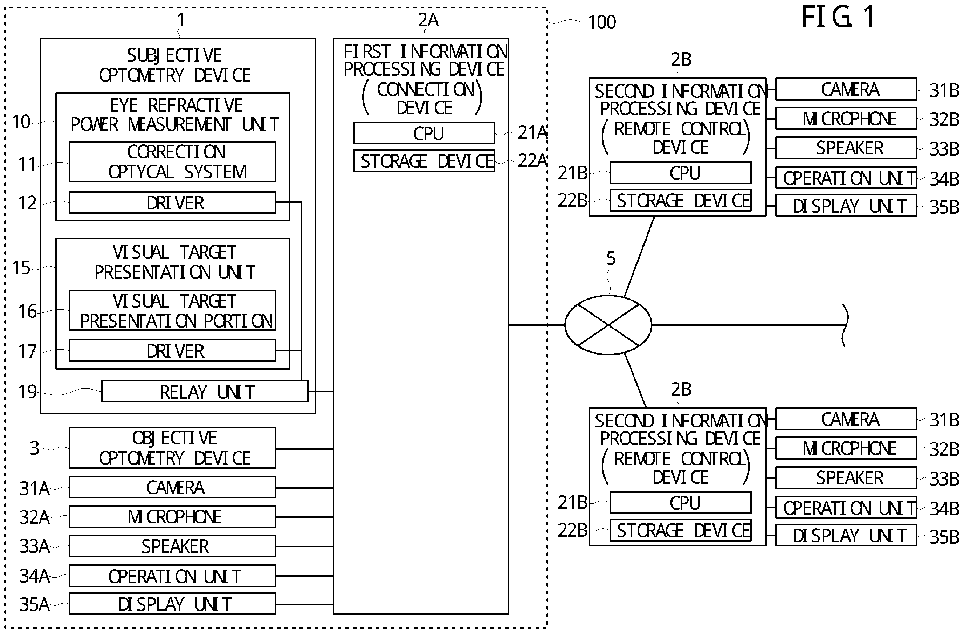

Here, an embodiment of the present disclosure will be described with reference to the drawings. As shown in FIG. 1, a subjective optometry system 100 of the present embodiment includes a subjective optometry device 1 and a first information processing device 2A. The subjective optometry device 1 is used to subjectively measure an optical characteristic of eyes of an examinee. The optical characteristic of the subject eye measured by the subjective optometry device 1 of this embodiment is a refractive power of the eyes. The measured refractive power may be at least one of the spherical power, cylindrical power, and astigmatism axis angle of the subject eye. The first information processing device 2A is connected to the subjective optometry device 1. Hereinafter, the first information processing device 2Amay also be referred to as a "connection device". Furthermore, the first information processing device 2A is connected to a second information processing device 2B, which is another information processing device, via a network 5. That is, the first information processing device 2A of this embodiment may be remotely accessed by the second information processing device 2B. Hereinafter, each device will be described in detail.

Description will be given regarding the subjective optometry device 1. The subjective optometry device 1 includes an eye refractive power measurement unit 10, a visual target presentation unit 15, and a relay unit 19.

The eye refractive power measurement unit 10 includes a correction optical system 11 and a driver 12. The correction optical system 11 changes the optical characteristic of a visual target light flux presented to the subject eye. Specifically, the correction optical system 11 changes at least one of the spherical power, cylindrical power, astigmatic axis angle, polarization characteristic, and aberration amount of the visual target light flux. For example, in this embodiment, the correction optical system 11 switchably selects an optical element to be arranged in an examination window in front of the subject eye among multiple optical elements to change the optical characteristics of the visual target light flux. In this embodiment, a lens disk for the left eye and a lens disk for the right eye, each having multiple optical elements arranged in a common circumferential direction, are used in the correction optical system 11. Each of the lens disks for the left eye and the right eye may be formed of a single lens disk or a plurality of lens disks.. Examples of the optical elements include, but are not limited to, spherical lenses, cylindrical lenses, cross-cylinder lenses, rotary prisms, and wavefront modulation elements. The driver 12 drives the correction optical system 11 to change the optical characteristics of the visual target light flux. In this embodiment, the driver 12 drives the correction optical system 11 to switch the optical elements in the examination window by rotating both the lens disks for the left eye and the right eye. A step motor or the like may be used for the driver 12. The driver 12 operates in response to a driving signal.

The visual target presentation unit 15 presents a visual target (for example, a Landolt ring or at least one of characters) to the subject eye and switches visual targets presented to the subject eye. Specifically, the visual target presentation unit 15 includes a visual target presentation portion 16 and a driver 17. The visual target presentation portion 16 presents one of the visual targets to the subject eye. Examples of the visual target presentation portion 16 include a space-saving visual target projecting device that projects a visual target onto the subj ect eye via a concave mirror, a chart projector that projects the visual target onto a screen, and a display that displays the visual target. The visual target presentation portion 16 is positioned to be away a particular distance (in a meaning of an optical system) from the subject eye and is placed at the same height as the eye refractive power measurement unit 10. The driver 17 drives the visual target presentation portion 16 to switch the inspection visual targets presented to the subject eye. The driver 17 operates in accordance with driving signals.

The relay unit 19 relays driving signals between the first information processing device 2A and the drivers 12, 17. In this embodiment, a system for the driving signals output from the first information processing device 2A and a system for the driving signals for controlling at least one of the drivers 12 and 17 are different. The relay unit 19 in this embodiment converts the driving signals received from the first information processing device 2A into driving signals for controlling the drivers 12 and 17, and transmits them to the drivers 12 and 17. Furthermore, in one example of this embodiment, when the relay unit 19 receives one driving signal from the first information processing device 2A so as to drive both the driver 12 and the driver 17 together, the relay unit 19 converts the received one driving signal into two driving signals to drive both the drivers 12 and 17, and transmits them to the drivers 12 and 17.

The first information processing device 2A and the second information processing device 2B will now be described. The first and second information processing devices 2A and 2B can be any of various information processing devices capable of processing various types of information. For example, personal computers (referred to as "PCs" hereinafter) may be used as the first and second information processing devices 2A and 2B in this embodiment. However, information processing devices that serve as the first and second information processing devices 2A and 2B in this embodiment are not necessarily limited to the PCs. For example, a server, mobile terminal, or smartphone may be used as at least one of the first and second information processing devices 2A and 2B. At least one of the first and second information processing devices 2A and 2B may be formed of multiple devices. For example, the first information processing device 2A may be formed of a personal computer and a dedicated controller having a controller and storage device.

The first information processing device 2A and the second information processing device 2B are communicatably connected to each other via the network (e.g., the Internet) 5. An example shown in FIG. 1 is a case where a plurality of second information processing devices 2B are connected to the single first information processing device 2A. However, one second information processing device 2B may be connected to one first information processing device 2A. Additionally, one second information processing device 2B may be connected to a plurality of first information processing devices 2A.

The first information processing device 2A is installed at a location where a subjective optometry is conducted for an examinee (e.g., an eyeglasses store or hospital). The first information processing device 2A is equipped with a CPU 21A and a storage device 22A. The CPU 21A is a control unit (controller) that controls the first information processing device 2A. The storage device 22A stores programs and various types of data. In this embodiment, the optometry control program is stored in the storage device 22A.

The first information processing device 2Ais communicatably connected to the subjective optometry device 1 (more specifically, to the relay unit 19 of the subjective optometry device 1). Various standards, such as LAN, can be used for connection between the first information processing device 2A and the subjective optometry device 1. Additionally, the first information processing device 2Ais connected to an objective optometry device 3. The objective optometry device 3 measures optical characteristics of the subject eye (such as spherical power, cylindrical power, and astigmatic axis angle) objectively. Various standards such as LAN can be used for connection between the first information processing device 2A and the objective optometry device 3. Note that the objective optometry device 3 may also be connected to the relay unit 19. The measurement results obtained from the objective optometry device 3 can be stored in a storage device of the relay unit 19.

The first information processing device 2Ais connected to a camera 31A, a microphone 32A, a speaker 33A, an operation unit 34A, and a display unit 35A. The camera 31A captures images. Specifically, in this embodiment, the camera 31A is used to capture moving images of the examinee. The microphone 32A converts sound into audio signals and outputs them. The speaker 33A converts audio signals into sound. The operation unit 34Ais operated by a user (such as an examinee) to input various instructions. For the operation unit 34A, at least one of a keyboard, a mouse, and a touch panel can be used, or a dedicated operation unit (such as a joystick) suitable for inputting responses for the subjective optometry can be used. The display unit 35A displays various images. Various devices capable of displaying images (such as a monitor, a display, and a projector) can be used as the display unit 35A.

Each of the second information processing devices 2B is located in a facility with an examiner (for example, an eyewear store employee who is knowledgeable in optometry using the subjective optometry device 1, a doctor, or a nurse, etc.) who is capable of performing an optometry using the subjective optometry device 1. Each of the second information processing devices 2B includes a CPU 21B and a storage device 22B. The CPU 21B is a control unit (controller) that controls the second information processing device 2B. The storage device 22B is capable of storing programs and various data, etc.

A camera 31B, a microphone 32B, a speaker 33B, an operation unit 34B, and a display unit 35B are connected to the second information processing device 2B. Various devices can be used as these devices, similar to the devices connected to the first information processing device 2A as described above.

### (Application - Optometric Method)

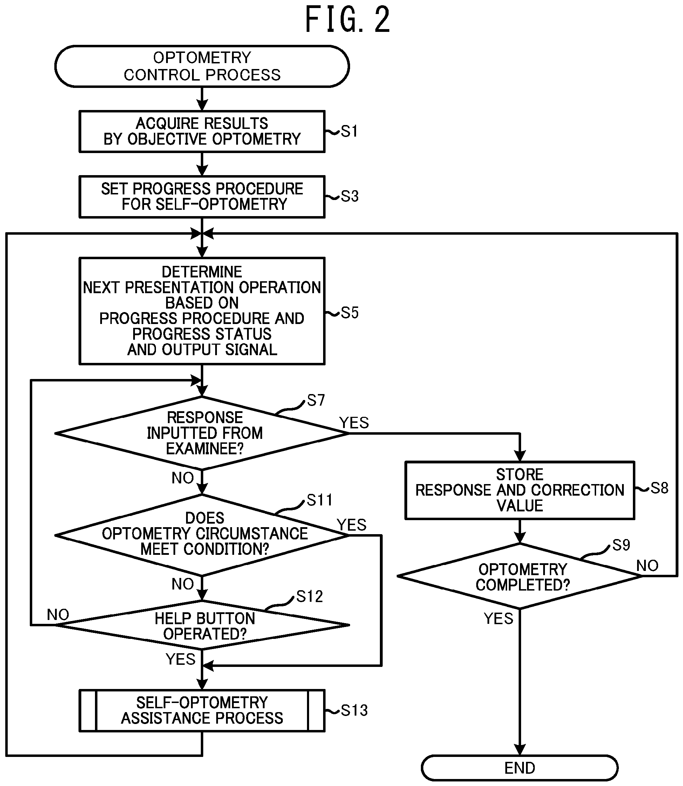

An application installed in the first information processing device 2A of this embodiment will now be described. As described above, the optometry control program for executing an optometry control process (refer to FIG. 2) is stored in the storage device 22A of the first information processing device 2A. The optometry control program includes a drive control application program for executing a drive control application and a self-optometry application program for executing a self-optometry application. The drive control application controls operation of the subjective optometry device 1 by transmitting control signals to the subjective optometry device 1. The self-optometry application automatically conducts the optometry by the subjective optometry device 1 based on responses inputted from an examinee. The drive control application program and the self-optometry application program may be separately formed and prepared, or may be incorporated into a single program.

Herein described is a subjective optometry method that can be performed by the subjective optometry system 100 in the present embodiment. The subjective optometry system 100 of the present embodiment can perform both a self-optometry and a remote-optometry. The self-optometry is an eye examination performed by the self-optometry application. In other words, during the self-optometry, the eye examination proceeds automatically based on responses inputted by an examinee. Furthermore, in the subjective optometry system 100 of the present embodiment, if a problem with the progress of the self-optometry occurs, a self-optometry assistance process is executed to assist the examinee in the progress of the self-optometry. The self-optometry assistance process can also be executed in response to an instruction signal inputted to one of the second information processing devices 2B that is located in a remote location. Therefore, even if a problem with the progress of the self-optometry occurs, the eye examination can smoothly continue. On the contrary, the remote-optometry is an eye examination performed in response to an instruction signal inputted to the second information processing device 2B. In the following description, the self-optometry will be mainly described.

### (Optometry control process)

Referring to FIGS. 2 to 4, an example of the optometry control process performed by the first information processing device 2A of the subjective optometry system 100 of the present embodiment will be described. The optometry control process includes processing for controlling the self-optometry, processing for assisting in the self-optometry, and the like. When an instruction to start a subjective optometry on an examinee is inputted to the first information processing device 2A, the CPU 21A of the first information processing device 2A executes the optometry control process as illustrated in FIG. 2 according to the optometry control program.

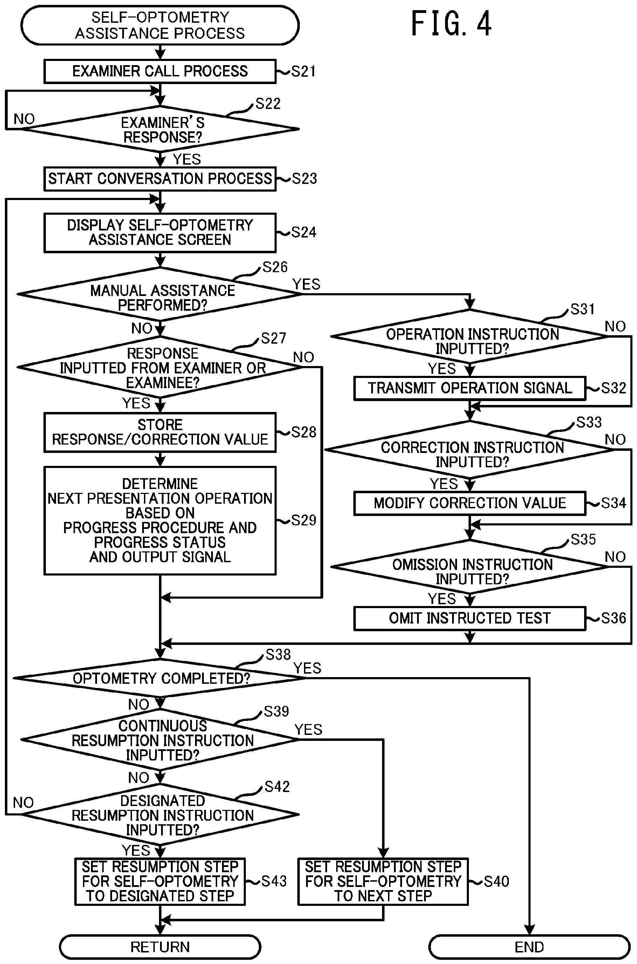

As an example, the optometry control process of the present embodiment is performed when communication (e.g., remote access) between one or more second information processing devices 2B and the first information processing device 2A has been established. However, communication between the first information processing device 2A and one or more of the second information processing devices 2B may also be established during the execution of the optometry control process (e.g., at the timing of starting a self-optometry assistance process illustrated in FIG. 4). The communication method between the first information processing device 2A and the second information processing device 2B may also be appropriately selected. For example, remote access service (RAS) can be used to establish the remote access of the second information processing device 2B to the first information processing device 2A.

First, the CPU 21A acquires results of an externally-conducted objective optometry (S 1). For example, in this embodiment, a LAN or the relay unit 19 is used to connect to the external objective optometry device 3 (refer to FIG. 1) to acquire the results of externally-conducted objective optometry for a same examinee. However, the CPU 21A may also acquire results of the externally-conducted objective optometry for the same examinee using, for example, a detachable memory or the network 5. Additionally, results of the externally-conducted objective optometry may also be inputted by the user via the operation unit 34A, etc. If there are no results of the externally-conducted objective optometry for the same examinee, the process at S1 may be skipped.

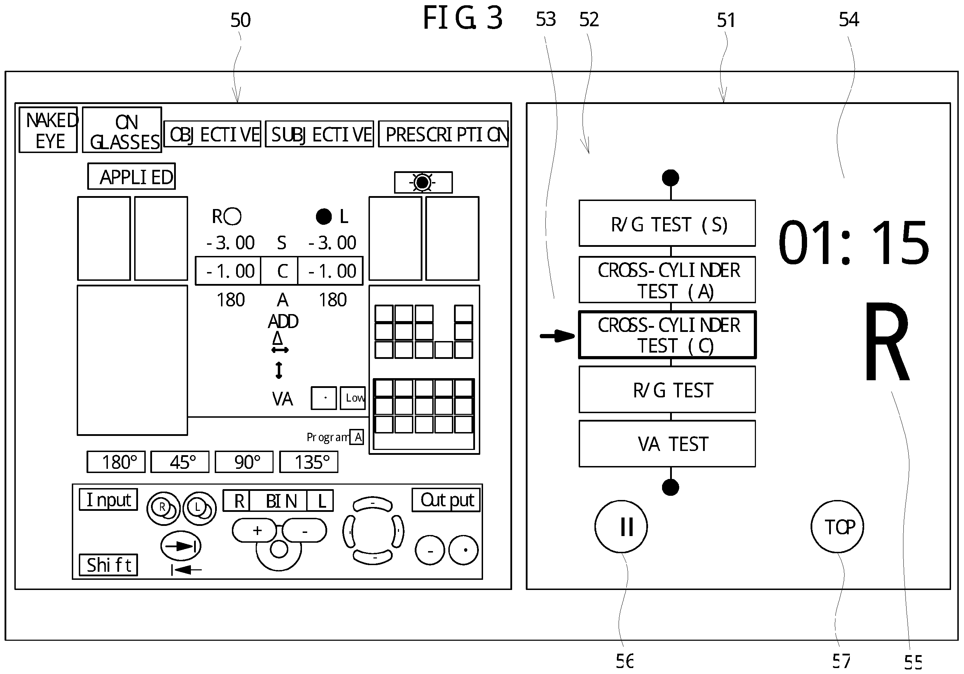

Next, the CPU 21A sets a progress procedure for the self-optometry (S3). An example of a part of the progress procedure for the self-optometry used in the subjective optometry system 100 of this embodiment is displayed in a progress procedure display field 52 shown in FIG. 3 (the progress procedure for the right eye is only shown in FIG. 3). As shown in FIG. 3, the progress procedure used in this embodiment sets (i) multiple tests (multiple examinations) executed for the examinee and (ii) the order in which these tests are executed. For example, in the progress procedure shown in FIG. 3, after executing R/G test (S), cross-cylinder test (A), cross-cylinder test (C), R/G test, and VA test for the right eye of the examinee, the same tests are executed for the left eye of the examinee in the following order: R/G test (S), cross-cylinder test (A), cross-cylinder test (C), R/G test, and VA test. In the R/G test (S), the spherical power of the subject's eye is measured. In the cross-cylinder test (A), the axis angle of astigmatism of the subj ect's eye is measured. In the cross-cylinder test (C), the degree of astigmatism of the subject's eye is measured. In the subsequent R/G test, it is confirmed whether an adjustment function of the subject's eye properly worked for the executed tests. In the VA test, the maximum visual acuity of the subject's eye is measured.

At S3, if the results of the objective optometry as to the same examinee were obtained at S1, the progress procedure for the self-optometry is set based on the results of the objective optometry (e.g., the refractive power measured for the subject eye (i.e., spherical power, astigmatic degree, and astigmatic axis angle)). For example, in each test included in the procedure, the type and size of the optical element to be first placed in the examination window of the correction optical system 11 and the type of the visual target to be presented on the display unit 16 may be set based on the results of the objective optometry. If the astigmatic degree obtained by the objective optometry falls below a threshold value, a procedure without the test related to astigmatism (e.g., the cross-cylinder test (A) and the cross-cylinder test (C)) may be set. At S3, when the results of the objective optometry are not obtained at S1, a default process may be set.

Next, the CPU 21A determines the presentation operation of the visual target to be executed next time by the subjective optometry device 1 based on the progress procedure set at S3 and the progress status of the self-optometry and outputs a presentation instruction signal (i.e., a driving signal) for executing the determined presentation operation to the subjective optometry device 1 (S5). Specifically, at S5 of this embodiment, at least one of the optical elements to be placed in the examination window of the correction optical system 11 and the visual target to be presented on the visual target presentation portion 16 is determined as the next presentation operation. A driving signal for at least one of the drivers 12 and 17 is transmitted to the subjective optometry device 1 to execute the determined operation. Note that when transmitting the driving signal to the subj ective optometry device 1, the CPU 21A outputs a guidance voice corresponding to examination contents from the speaker 33A. Therefore, the examinee will see the presented visual target after appropriately understanding the contents of the examination.

In the present embodiment, the driving signal is transmitted from the first information processing device 2Ato the drivers 12 and 17 through the relay unit 19 described above (see FIG. 1). Therefore, there is no need for a dedicated controller through which signals transmitted from the first information processing device 2A pass. Thus, signal processing by such a dedicated controller can be omitted, leading to smoother execution of the optometry.

In the self-optometry, the examinee understands the test contents through the guidance audio, visually recognizes the presented visual target displayed on the subjective optometry device 1, and enters its response to the visually-recognized visual target into the first information processing device 2A. As an example, in the present embodiment, the dedicatedly-manufactured operation unit 34A suitable for entering responses for the subjective optometry is operated by the examinee to input his/her responses. However, responses may also be input using a general-purpose operation unit 34A, such as a mouse, touch panel, or keyboard. Responses may also be inputted using voice signals converted by the microphone 32A.

The CPU 21A determines whether a response has been inputted from the examinee (S7). When a response has been inputted (S7: YES), the input response and the optical correction value (measurement value) for the specific optical characteristics of the subject eyes are stored in the storage device 22A (S8). The optical correction value for the optical characteristics of the subject eyes is acquired based on the input response at S7, the visual target presented to the examinee when the response was made, and the optical characteristics of the optical element (i.e., the optical characteristics of the visual target light flux) set within the examination window of the correction optical system 11.

If a series of self-optometry processes has not yet been completed (S9: NO), the process returns to S5 and the self-optometry continues in accordance with the progress procedure. For example, in the VA test of this embodiment, if the response from the examinee obtained at S7 is correct, the CPU21A determines the next visual target presentation operation so that the visual target to be presented next by the visual target presentation portion 16 is an visual target with a visual acuity value one step higher than the previously presented visual target (e.g., a visual target being one step smaller in size). Furthermore, if the response from the examinee obtained at S7 is incorrect, CPU21A determines the next visual target presentation operation so that the visual target presented by the visual target presentation portion 16 is a visual target with a visual acuity value one step lower than the previously presented visual target (e.g., a visual target being one step larger in size). Also, along with switching of the visual target, the CPU21A may determine, as the next visual presentation operation, the correction degree of the optical element placed in the examination window of the correction optical system 11.

Note that if the processes at S7 to S9 are repeatedly executed and the entire self-optometry is successfully completed (S9: YES), the optometry control process ends.

If no response is inputted from the examinee (S7: NO), it is determined (S11) whether a predetermined condition that indicates the self-optometry inappropriately proceeds is met. For example, in this embodiment, when a predetermined time has elapsed without a response being inputted from the examinee since the presentation instruction signal was transmitted at S5, the predetermined condition is determined to be met. Furthermore, even if the response inputted by the examinee is inappropriate (e.g., a response other than requested candidate responses is inputted, the same response is inputted continuously more than a predetermined number of times, or more responses than the number requested are inputted), the predetermined condition is determined to be met. If it is determined that the predetermined condition is met (S1 1: YES), the self-optometry assistance process is executed (S13).

If the response from the examinee is not inputted (S7: NO) and the condition for the self-optometry is not met (S11: NO), the CPU 21A determines whether an instruction for executing an assistance operation for the self-optometry has been inputted by the examinee (S12). For example, in this embodiment, the examinee can input the instruction for executing the assistance operation for the self-optometry by operating a help button provided on the dedicated controller or a help button image visually displayed on the display unit 35A. If the instruction is not inputted (S12: NO), the process returns to S7. If the instruction for executing the assistance operation is inputted (S12: YES), the self-optometry assistance process is executed (S13).

Referring to FIG. 4, the details of the self-optometry assistance process will be described. First, the CPU 21A performs an examiner call process (S21). For example, at S21 of this embodiment, the CPU 21A transmits an examiner call instruction via the network 5 to one or more second information processing devices 2B for which remote access to the first information processing device 2A has been established (S4). The examiner call instruction is an instruction for causing the second information processing device 2B to perform a calling action to request an examiner for assistance in the self-optometry. The calling action may be performed by at least one of, for example, outputting sound or displaying an image. By performing the calling action, the user (examiner) of the second information processing device 2B can appropriately understand that assistance in the self-optometry (remote assistance in this embodiment) is requested.

Next, the CPU 21A determines whether there is a response from the examiner, who is a user of the second information processing device 2B (S22). If there is no response from the examiner from any one of the second information processing devices 2B (S22: NO), assistance by the examiner in the self-optometry is not currently available, so the determination process at S22 is repeated and the system waits. If a user of one of the second information processing devices 2B is in a state where he/she can assist in the self-optometry and inputs a response instruction to the one of the second information processing devices 2B (S22: YES), the process proceeds to S23.

Next, the CPU 21A starts communication by transmitting and receiving voice data between the examiner using the second information processing device 2B and the system, thus initiating a conversation process (S23). As a result, the examiner and the examinee can talk, and the assistance process of the self-optometry described below is executed. Specifically, the CPU 21A transmits voice data inputted from the microphone 32A to the second information processing device 2B. In addition, the CPU 21A receives the voice data inputted from the second information processing device 2B through the microphone 32B and outputs it to the speaker 33A. Note that the CPU 21A may also transmit and receive image data to/from the second information processing device 2B along with the voice data. In this case, the CPU 21A may transmit the image data inputted from the camera 31A to the second information processing device 2B. In addition, the CPU 21A may receive the image data inputted from the second information processing device 2B through the camera 32B and display it on the display unit 35A.

Next, the CPU 21A displays a self-optometry assistance screen image (see Fig. 3) on the display unit 35B of the second information processing device 2B used by the examiner who is assisting in the self-optometry (S24). As shown in FIG. 3, the self-optometry assistance screen image in this embodiment includes an operation image area 50 and a progress procedure display area 51.

In the operation image area 50, an operation image including information on the optical characteristics of the visual target light flux presented to the subject eye. In the operation image of this embodiment, values relating to the optical characteristics of the visual target light flux presented to the subject eye are displayed for each type of optical characteristic. The examiner can indicate the value for the desired type of optical characteristic (spherical power, cylindrical power, and astigmatic axis angle, etc.) by operating various buttons and values on the operation image area 50 through an operation device such as a touch panel or a mouse. When the optometry is performed properly, a correction value of the optical characteristic displayed on the operation image area is a measured value of the optical characteristic of the subject eye.

In this embodiment, the progress procedure display area 51 is displayed not only on the display unit 35B connected to the second information processing device 2B but also on the display unit 35A connected to the first information processing device 2A. The progress procedure display area 51 in this embodiment includes the progress procedure display field 52, an elapsed time display field 54, a left/right eye display filed 55, a help button 56, and a top button 57. As mentioned above, the progress procedure set for the ongoing self-optometry is displayed in the progress procedure display field 52. In the example shown in FIG. 3, the test currently being performed among the entire progress procedure (i.e., multiple tests included in the progress procedure) is indicated by an arrow 53 and is emphasized by a bold frame.

The elapsed time display field 54 displays elapsed time since the start of the ongoing self-optometry. The left/right eye display field 55 displays which eye (left or right) of the examinee at the currently performed step among the entire progress procedure is a targeted eye for examination. The help button 56 is operated by the examinee to input an instruction for executing the assistance operation of the self-optometry. The top button 57 is operated to return the screen displayed in the progress procedure display area 51 to the initial page of the self-optometry application.

During the assistance operation of the self-optometry described hereinafter (S24-S43), as mentioned above, the voice signal inputted from the microphone 32A to the first information processing device 2A is converted into sound by the speaker 33B of the second information processing device 2B. Similarly, the voice signal inputted from the microphone 32B to the second information processing device 2B is converted into sound by the speaker 33A of the first information processing device 2A. Therefore, the examiner and the examinee can communicate during the assistance operation of the self-optometry. In addition, during the assistance operation of the self-optometry, the examiner using the second information processing device 2B can input various instructions to assist in operation of the self-optometry using at least one of the operation unit 34B and the microphone 32B.

Next, the CPU 21A determines whether manual assistance by the examiner is being performed (S26). In this embodiment, either indirect assistance or manual assistance is selected as a method for assisting in operation of the self-optometry by the examiner. The indirect assistance is a method in which the examiner indirectly assists in the self-optometry while the self-optometry by the self-optometry application is being performed in accordance with the progress procedure set in S3. The manual assistance is a method for assisting in operation of the self-optometry by directly advancing the examination in response to instructions inputted by the examiner instead of following the self-optometry procedure.

The examiner can control the first information processing device 2A to execute either the indirect assistance or the manual assistance in various ways. For example, in this embodiment, the indirect assistance is set to be executed first when the self-optometry assistance process starts. In addition, the indirect assistance may be switched to the manual assistance by inputting an operation instruction, a correction instruction for modifying the correction value, or an omitting instruction for omitting the procedure into the subjective optometry device 1 through various buttons or the like displayed in the operation image area 50. However, the method for switching from the indirect assistance to the manual assistance can be appropriately selected. For example, a button for switching between the indirect assistance and the manual assistance may be provided on the self-optometry assistance screen.

When the indirect assistance is being performed (S26: NO), the CPU 21A determines whether a response by the examinee who visually recognized the presented visual target has been inputted by the examiner or the examinee (S27). In this embodiment, during the execution of the indirect assistance, the examiner can input the response heard from the examinee into the second information processing device 2B by operating the operation unit 34B connected to the second information processing device 2B. The response inputted to the second information processing device 2B is transmitted to the first information processing device 2A. Therefore, even if the examinee is not familiar with a way of responding for the self-optometry, assisting in the self-optometry can be appropriately performed. In addition, even during the execution of the indirect assistance in this embodiment, as described above, the examinee can also input his/her response by himself/herself (see FIG. 2). Therefore, once the examinee understands the way of responding from an advice by the examiner, it is also possible for the examinee to input the response himself/herself and proceed with the optometry. However, during the execution of the indirect assistance, either the first information processing device 2A or the second information processing device 2B may accept the response.

If the response by the examinee is not inputted into either the first information processing device 2A or the second information processing device 2B (S27: NO), the process proceeds to S38. When the response of the examinee is inputted into the first information processing device 2A or the second information processing device 2B (S27: YES), the inputted response and the optical measurement value of the subject eye are stored in the storage device 22A, similar to S8 (see FIG. 2) (S28). Then, the CPU 21A determines the presentation operation of the visual target to be executed next at the subjective optometry device 1 according to the progress procedure set at S3 (see FIG. 2) and the progress status at that time, and outputs a presentation instruction signal (driving signals) for executing the determined presentation operation to the subjective optometry device 1 (S29). That is, the subjective optometry in accordance with the progress procedure executed by the subjective optometry application continues thereafter. After that, the process proceeds to S38.

Furthermore, if the manual assistance is being executed instead of the indirect assistance (S26: YES), the CPU 21A determines whether an operation instruction for allowing the examiner to manually proceed with at least a part of the progress procedure has been inputted by the examiner (S31). The examiner can determine the operation instruction for the self-optometry device 1, such as an instruction for an arrangement operation of the optical element in the correction optical system 11 and an instruction for a display operation of the visual target in the display unit 16, based on the contents displayed on the self-optometry assistance screen image (see FIG. 3) or the conversation with the examinee. In this case, the examiner inputs the determined operation instruction to the second information processing device 2B via the operation unit 34B or the like. The inputted operation instruction is acquired by the first information processing device 2A via the network 5. When the operation instruction is inputted and acquired (S31: YES), the CPU 21Atransmits a display instruction signal (i.e., a driving signal) to the subjective optometry device 1 to execute the instructed operation (S32).

Next, the CPU 21A determines whether an instruction for modifying the correction value stored at S8 (see FIG. 2) or S28 by the self-optometry has been inputted (S33). If the examiner determines that the correction value (i.e., the measured value) displayed on the self-optometry assistance screen image (see FIG. 3) needs to be modified, the examiner inputs a modification instruction for the correction value necessary for modification to the second information processing device 2B via the operation unit 34B or the like. The inputted modification instruction is acquired by the first information processing device 2A via the network 5. When the modification instruction is inputted and acquired (S33: YES), the CPU 21A modifies the correction value according to the instruction (S34).

Then, the CPU 21A determines whether an omission instruction for omitting at least a part of the progress procedure (in this embodiment, at least one of multiple tests) has been inputted (S35). When the omission instruction is inputted via the second information processing device 2B and the network 5 (S35: YES), the CPU 21A omits the instructed test from the progress procedure (S36). Then, the process proceeds to S38.

Next, the CPU 21A determines whether a series of the optometry for the examinee has been completed (S38). For example, the CPU 21A may determine that the series of the optometry has been completed if the self-optometry performed according to the progress procedure has been completed. Alternatively, the CPU 21A may determine that the series of the optometry has been completed when an instruction to end the optometry for the examinee has been inputted. When the series of the optometry has been completed (S38: YES), the process ends.

Next, the CPU 21A determines whether a continuous resumption instruction for the self-optometry has been inputted (S39). The continuous resumption instruction refers to an instruction to resume the self-optometry from the next step after the step for which the optometry has already been completed among the progress procedure set at S3 (see FIG. 2). For example, the CPU 21A may determine that the continuous resumption instruction has been inputted if a continue button (not shown) has been operated by the examinee. When the continuous resumption instruction is inputted via the second information processing device 2B and the network 5 (S39: YES), the CPU 21A sets a resumption step for the self-optometry as a next step subsequent to the step for which the optometry has already been completed among the progress procedure (S40), and the process returns to the optometry control process (see FIG. 2). In the processing of S5 to S9 that follows, the self-optometry is resumed from the resumption step set in S40. When the instruction to continue is not inputted (S39: NO), the process proceeds to S42.

Next, the CPU 21A determines whether a designated resumption instruction for the self-optometry has been inputted (S42). The designated resumption instruction is an instruction to resume the self-optometry from a step designated by the examiner among the progress procedure set at S3 (see Fig. 2). In this embodiment, the examiner can input the designated resumption instruction by designating the resumption step for resuming the self-optometry on the progress procedure displayed in the progress procedure display field 52 in the self-optometry assistance screen image (see Fig. 3). For example, in this embodiment, the examiner can input the designated resumption instruction using a mouse or a touch panel. Note that a designated resumption instruction button (i.e., a reset button) for removing the results of the self-optometry that had been done according to the process procedure and restarting the self-optometry from the first step of the progress procedure may be disposed in the self-optometry assistance display image. When the designated resumption instruction is inputted via the second information processing device 2 and the network 5 (S42: YES), the CPU 21A sets the step designated by the designated resumption instruction among the progress procedure as the resumption point of the self-optometry (S43), and the process returns to the optometry control process (see Fig. 2). In the subsequent processing of S5 to S9, the self-optometry is resumed from the restart step set in S43.

The above-described disclosure in the above embodiment is just one example. Therefore, it is also possible to modify the technology illustrated in the above embodiment. For example, it is possible to execute only a part of the technology illustrated in the above embodiment. The subjective optometry system 100 in the above embodiment executes both the examiner call process (S21) and the self-optometry assistance process by the examiner (S23 to S43) when a problem occurs during the self-optometry (S11: YES or S12: YES). However, the subjective optometry system 100 may execute only one of the examiner call process and the assistance process by the examiner. For example, even if the examiner is called by only executing the examiner calling process, assisting in the self-optometry can be appropriately performed.

In the above embodiment, the self-optometry starts with the second information processing device 2B being connected to the first information processing device 2A. However, after a problem has occurred during the self-optometry (S11: YES or S12: YES), the second information processing device 2B may be connected to the first information processing device 2A.

The self-optometry assistance process of this embodiment (see FIG. 4) is executed in response to the instructions inputted to the second information processing device 2B located remotely. However, the self-optometry assistance process may also be executed in response to an instruction directly inputted to the first information processing device 2A (for example, an instruction inputted to the first information processing device 2Aby the operation unit 34A operated by an examiner, etc.). In other words, an examiner at a location where the self-optometry device 1 is installed may input an instruction to assist in the self-optometry. Moreover, both the first information processing device 2A and the second information processing device 2B may accept input of instructions for assisting in the self-optometry. In this case, the examiner can assist in the self-optometry at both the location where the self-optometry device 1 is installed and a remote location. Note that when instructions for assisting in the self-optometry are inputted via the first information processing device 2A, the process of calling for the examiner at S21 (see FIG. 4) may be executed at the first information processing device 2A or may be executed at both the first information processing device 2A and the second information processing device 2B.

Note that at S5 of FIG. 2 and SS29 of FIG. 4, the process of outputting the presentation instruction signal to the self-optometry device 1 based on the progress procedure is an example of the "self-optometry progress step." The process of obtaining a response from the examinee at S7 of FIG. 2 and S27 of FIG. 4 is an example of a "response acquisition step." The process of storing a correction value in S8 of FIG. 2 and S28 of FIG. 4 is an example of a "correction value storing step." The self-optometry assistance process shown in FIG. 4 is an example of an "self-optometry assistance step." The process of outputting a presentation instruction signal in response to the operation instruction at S31 and S32 of FIG. 4 is an example of a "manual progress step." The process of modifying the correction value at S33 and S34 of FIG. 4 is an example of a "correction value modification step." The process of omitting progress steps at S35 and S36 of FIG. 4 is an example of a "procedure omission step." The process of obtaining the examinee's response in response to instructions inputted by the examiner at S27 of FIG. 4 is an example of a "proxy response acquisition step". The processing for resuming the self-optometry at S39 and S40 of FIG. 4 is an example of a "continuous resumption step". The processing for resuming the self-optometry according to the step specifically designated by the examiner at S42 and S43 of FIG. 4 is an example of a "designated resumption step". The processing for resuming the self-optometry at S33 and S34 of FIG. 4 is an example of an "self-optometry resumption step". The processing for displaying the progress procedure at S24 ofFIG. 4 is an example of a "procedure display step".

## Claims

1. An optometry control program for a subjective optometry system including a subjective optometry device and a first information processing device connected to the subjective optometry device, the subjective optometry device including a correction optical system that changes an optical characteristic of a visual target light flux presented to a subject eye and a visual target presentation unit that is configured to present a visual target to the subject eye, the subjective optometry device subjectively measuring an optical characteristic of the subject eye, the program being executed by the first information processing device and comprising:
a self-optometry application program that realizes an application for causing a self-optometry to proceed automatically based on a response from an examinee, wherein
the self-optometry application program, when executed by a controller of the first information processing device, causes the first information processing device to perform:
a self-optometry progress step of outputting, to the subjective optometry device, a presentation instruction signal for performing a presentation operation to present the visual target to the examinee in accordance with a progress procedure for the self-optometry that automatically proceeds;
a response acquisition step of acquiring the response from the examinee who visually recognized the presented visual target;
a correction value storing step of storing a correction value of the optical characteristic of the subject eye, the correction value being acquired based on the response acquired at the response acquisition step, the visual target that was presented by the subjective optometry device when the response was acquired, and the optical characteristic of the visual target light flux; and
a self-optometry assistance step of executing an assistance operation to assist in a progress of the self-optometry when a problem occurs during the progress of the self-optometry.

2. The optometry control program according to claim 1, wherein
during the self-optometry assistance step, the assistance operation to assist in the progress of the self-optometry is executed when a circumstance of the self-optometry in operation satisfies a predetermined condition or when an instruction for executing the assistance operation is inputted.

3. The optometry control program according to claim 1 or 2, wherein
the assistance operation executed at the self-optometry assistance step includes a call operation for calling for an examiner.

4. The optometry control program according to any one of claims 1 to 3, wherein
the self-optometry assistance step further includes a manual progress step of outputting, to the subjective optometry device, the presentation instruction signal to proceed with at least a part of the progress procedure in response to an instruction inputted by an examiner.

5. The optometry control program according to any one of claims 1 to 4, wherein
the self-optometry assistance step further includes a correction value modification step of modifying, in response to an instruction inputted from an examiner, at least one of correction values stored during the self-optometry.

6. The optometry control program according to any one of claims 1 to 5, wherein
the self-optometry assistance step further includes a procedure omission step of omitting at least a part of the progress procedure in response to an instruction inputted from an examiner.

7. The optometry control program according to any one of claims 1 to 6, wherein
the self-optometry assistance step further includes a proxy response acquisition step of acquiring, in response to an instruction inputted from an examiner, a response from the examinee who visually recognized the visual target that was presented to the examinee in accordance with the progress procedure at the self-optometry progress step.

8. The optometry control program according to any one of claims 1 to 7, wherein
the self-optometry assistance step further includes a continuous resumption step of resuming the self-optometry, which was temporarily stopped, from a step among the progress procedure that is next to a step for which the optometry has been already completed.

9. The optometry control program according to any one of claims 1 to 8, wherein
the self-optometry assistance step further includes a designated resumption step of resuming the self-optometry, which was temporarily stopped, from a step among the progress procedure that is designated by an examiner.

10. The optometry control program according to claim 9, wherein
the self-optometry assistance step further includes a procedure display step of displaying the progress procedure on a display unit, and
at the designated resumption step, an instruction for designating the step from which the self-optometry resumes is inputted through the progress procedure displayed on the display unit.

11. The optometry control program according to any one of claims 1 to 10, wherein
the self-optometry assistance step is executed in response to an instruction inputted into a second information processing device that is another information processing device connected to the first information processing device via a network.

12. A subjective optometry system, comprising:
a subjective optometry device including a correction optical system that changes an optical characteristic of a visual target light flux presented to a subject eye and a visual target presentation unit that is configured to present a visual target to the subject eye, the subjective optometry device subjectively measuring an optical characteristic of the subject eye; and
a first information processing device connected to the subjective optometry device, wherein
the first information processing device includes a self-optometry application program that causes a self-optometry to proceed automatically based on a response from an examinee,
the self-optometry application program, when executed, performs:
a self-optometry progress step of outputting, to the subjective optometry device, a presentation instruction signal for performing a presentation operation to present the visual target to the examinee in accordance with a progress procedure for the self-optometry that automatically proceeds;
a response acquisition step of acquiring the response from the examinee who visually recognized the presented visual target;
a correction value storing step of storing a correction value of the optical characteristic of the subject eye, the correction value being acquired based on the response acquired at the response acquisition step, the visual target that was presented by the subjective optometry device when the response was acquired, and the optical characteristic of the visual target light flux; and
a self-optometry assistance step of executing an assistance operation to assist in a progress of the self-optometry when a problem occurs during the progress of the self-optometry.
